# EUROPÄISCHE PATENTSCHRIFT

(11) **EP 2 640 303 B1**
(45) Veröffentlichungstag und Bekanntmachung des Hinweises auf die Patenterteilung: **04.01.2017**
(21) Anmeldenummer: 11779398.4
(22) Anmeldetag: 04.11.2011
(51) Int. Cl.: A61B 8/00, A61B 46/10, A61B 46/17

(54) **ABDECKUNG FÜR ULTRASCHALLKOPF**
COVER FOR AN ULTRASONIC HEAD
PROTECTION POUR UNE TÊTE À ULTRASONS

(30) Priorität: 19.11.2010 AT 7072010
(43) Veröffentlichungstag der Anmeldung: 25.09.2013
(73) Patentinhaber: Lackner, Leopold, 3370 Ybbs (AT)
(72) Erfinder: Lackner, Leopold, 3370 Ybbs (AT)
(74) Vertreter: KLIMENT & HENHAPEL
(86) Internationale Anmeldenummer: PCT/EP2011/069413
(87) Internationale Veröffentlichungsnummer: WO 2012/065859

(56) Entgegenhaltungen:
- WO-A2-2007/011689
- DE-A1-102007 007 742
- DE-U1-202007 003 259
- US-A- 3 698 791

## Beschreibung

Die Erfindung ist in Anspruch 1 definiert und betrifft eine Ultraschallkopfabdeckung in Form eines Folienschlauches, der sackähnlich zur Umhüllung eines handgeführten Ultraschallkopfes und seiner Kabelzuführung ausgeführt ist und im Aufbewahrungszustand eine verkürzende Faltung, die einen taschenförmigen Eingriffsbereich bildet, aufweist, wobei der Folienschlauch eine Klebefläche, die von einem ersten Kleber gebildet wird, zur Befestigung des Folienschlauches am Ultraschallkopf aufweist.

Handgeführte Ultraschällkopfgeräte werden in der Medizin zur Unterstützung der medizinischen Diagnostik und der Behandlung von Patienten eingesetzt, wobei die Schallfläche des Ultraschallkopfes, also die Austrittsfläche des Ultraschalls am Ultraschallkopf, mit leichtem Druck auf dem Körper des Patienten geführt wird. Die Ultraschallwellen werden von im Schallkopf eingelagerten speziellen Kristallen mittels des piezoelektrischen Effektes erzeugt. Hierbei regt eine hochfrequente elektrische Wechselspannung die Kristalle zu Schwingungen an, welche Druckschwankungen in Form von Ultraschall verursachen. Umgekehrt erzeugt eine auf dem Kristall auftreffende Ultraschallwelle eine messbare elektrische Spannung, welche schließlich vom Ultraschallgerät als Bildpunkt dargestellt wird. Der Schallkopf ist dabei in der Regel aus Hartplastik gefertigt, im Bereich der Austrittsfläche des Ultraschalls ist hingegen zumeist ein elastisches Material vorgesehen, wie etwa Kautschuk.

Wesentlich für eine aufschlussreiche Bildgebung ist dabei eine gute Schallübertragung von der Schallfläche in den Körper des Patienten. Zur Verbesserung der Schallübertragung bedient man sich in der Regel eines speziellen Gels, das auf den Körper des Patienten aufgetragen wird. Ultraschall breitet sich nämlich in flüssigen oder gelförmigen Medien sehr gut aus, in gasförmigen Medien hingegen sehr schlecht. Gasförmige Übertragungsmedien stellen somit optisch dichtere Bereiche dar, die somit zu unerwünschten Reflexionen und Beugungen des Ultraschalls führen, die die Bildgebung beeinträchtigen. Aus hygienischen Gründen wird der Schallkopf außerdem in der Regel mit einem Folienschlauch umhüllt, der auch steril ausgeführt sein kann. Da aber die bloße Umwicklung der Schallfläche mit einer Folie aufgrund von Faltenbildung ebenfalls zu Lufteinschlüssen führen kann, die die Qualität des Ultraschallbildes beeinträchtigen, ist auch zwischen dem Ultraschallkopf und dem Folienschlauch ein Gel aufzutragen, was in der täglichen Anwendung aber als mühsam und unangenehm empfunden wird,

Daher wurde auch vorgeschlagen, den Ultraschallkopf am Folienschlauch mittels einer vollflächigen Klebefläche zu befestigen. Somit kann ein faltenfreier Übergang zwischen der Folienabdeckung und der Schallfläche ohne Lufteinschlüsse sichergestellt werden, ohne dass eine zusätzliche Gelschicht zwischen Schallkopf und Folieninnenseite zur Schallübertragung notwendig wäre.

Allerdings stellt sich dabei das Problem, dass durch oftmaliges Anbringen und Ablösen der Klebefläche vom Schallkopf die Schallfläche beschädigt werden kann, etwa die Kautschukmembran, oder die schwingenden Piezo-Kristalle, was teure Reparaturen nach sich zieht. Außerdem verbleiben oft Rückstände des Klebers am Ultraschallkopf, die mitunter mühsam entfernt werden müssen, wobei auch die oftmalige Reinigung den Ultraschallkopf beschädigen kann.

Daher wurde in weiterer Folge auch vorgeschlagen, dass die Klebefläche ringförmig ausgeführt ist und eine kleberfreie Fläche umschließt, die in ihrer Ausdehnung im Wesentlichen der Austrittsfläche der Schallwellen am Ultraschallkopf entspricht, um Rückstände des Klebers an den sensiblen Bereichen des Ultraschallkopfes zu vermeiden. Bei einer solchen Ausführungsform hat sich jedoch herausgestellt, dass der Folienschlauch nicht ausreichend faltenfrei in Position gehalten werden kann, sodass der Übergang zwischen der Folienabdeckung und der Schallfläche nicht ohne Lufteinschlüsse sichergestellt werden kann.

Die Bildgebung erzielt somit keine zufrieden stellenden Resultate.

Weitere Folienschläuche unterschiedlichster Ausführungen wurden in der US 3 698 791, DE 20 2007 003 259 U1, WO 2007/011689 und DE 10 2007 007 742 beschrieben.

Es ist daher das Ziel der Erfindung, eine Ultraschallkopfabdeckung bereit zu stellen, die einerseits den Einsatz von Gel als Überträgermedium zwischen Schallkopf und Folienschlauch erübrigt, aber andererseits den Schallkopf weitestgehend schont, auch bei oftmaliger Verwendung einer Folienschlauchabdeckung. Des Weiteren sollen Rückstände des Klebers am Schallkopf weitestgehend vermieden werden. Diese Ziele werden durch die Merkmale von Anspruch 1 erreicht.

Anspruch 1 bezieht sich auf eine Ultraschallkopfabdeckung in Form eines Folienschlauches, der sackähnlich zur Umhüllung eines handgeführten Ultraschallkopfes und seiner Kabelzuführung ausgeführt ist und im Aufbewahrungszustand eine verkürzende Faltung, die einen taschenförmigen Eingriffsbereich bildet, aufweist, wobei der Folienschlauch eine Klebefläche, die von einem ersten Kleber gebildet wird, zur Befestigung des Folienschlauches am Ultraschallkopf aufweist. Hierbei ist vorgesehen, dass ein, mit dem Folienschlauch dicht verbundener Träger in Form einer Trägerfolie für die Klebefläche vorgesehen ist, der gute Durchlässigkeit gegenüber Schallwellen aufweist, und der Folienschlauch im Bereich der Klebefläche eine Schallöffnung in Form einer Aussparung aufweist, wobei der Träger im Durchtrittsbereich der Schallwellen angeordnet ist und die Schallöffnung abdeckt, und die Klebefläche vollflächig auf der, dem Ultraschallkopf zugewandten Seite des Trägers aufgetragen ist.

Es wäre zwar auch denkbar, einen Träger für die Klebefläche vorzusehen, der auf seiner, dem Schallkopf zugewandten Seite über einen ersten Kleber mit niedriger Haftkraft versehen ist, der somit beim Ablösen vom Ultraschallkopf vollständig auf dem Träger verbleibt, und auf der, dem Schallkopf abgewandten Seite einen zweiten Kleber mit vergleichsweise hoher Haftkraft, um den Träger sicher auf dem Folienschlauch zu befestigen. In diesem Fall wäre aber der Ultraschallkopf vom Körper des Patienten durch eine Abfolge von vier Schichten getrennt, nämlich der vollflächigen Klebeschicht des ersten Klebers niedriger Haftkraft, der Trägerschicht, der vollflächigen Klebeschicht des zweiten Klebers höherer Haftkraft, sowie der eigentliche Folienschlauch. Diese Abfolge von vier Schichten beeinträchtigt wiederum die Qualität der Bildgebung, wenngleich Rückstände des ersten Klebers auf dem Ultraschallkopf auf diese Weise erfolgreich unterbunden werden können.

Daher ist vorgesehen, den Folienschlauch mit einer Schallöffnung zu versehen, also einer Aussparung im Folienschlauch, um im Durchtrittsbereich der Schallwellen die durch den Folienschlauch gegebene Trennschicht zu vermeiden, und als Abdeckung in diesem Bereich den Träger der Klebefläche zu verwenden, der zu diesem Zweck dicht, in der Regel keimdicht, mit dem Folienschlauch verbunden wird. Auf diese Weise können von den oben erwähnten, vier Trennschichten zwei Trennschichten vermieden werden, nämlich die durch den Folienschlauch gegebene Schicht, sowie die Klebeschicht des zweiten Klebers. Mithilfe der Maßnahmen kann somit einerseits aufgrund der Verwendung eines geeigneten Trägers eine Klebefläche niedriger Haftkraft verwirklicht werden, die sich ohne Rückstände vom Ultraschallkopf ablösen lässt, und andererseits mithilfe der Schallöffnung eine gute Bildgebung erzielt werden. Des Weiteren ist kein zusätzliches Medium, etwa ein Gel zwischen Schallkopf und Folieninnenseite, zur Schallübertragung mehr notwendig.

Die dichte Verbindung zwischen dem Träger und dem Folienschlauch kann auf unterschiedliche Weise bewerkstelligt werden, etwa durch Verschweißen. Hierfür werden der Träger und der Folienschlauch jeweils aus einem thermoplastischen Kunststoff gefertigt sein, um ein Verschweißen des Trägers mit dem Folienschlauch zu ermöglichen. Eine Schweißverbindung zwischen zwei Kunststoffen stellt dabei in der Regel eine keimdichte Verbindung dar.

Alternativ wird vorgeschlagen, dass die Klebefläche zur Befestigung des Folienschlauches am Ultraschallkopf von einem ersten Kleber gebildet wird, und der Träger mithilfe eines zweiten Klebers mit dem Folienschlauch dicht verbunden ist. Diese Ausführung ermöglicht eine besonders einfache und kostengünstige Herstellung des erfindungsgemäßen Folienschlauches.

Wie noch näher beschrieben wird, bestehen grundsätzlich zwei Möglichkeiten für die Befestigung des Trägers am Folienschlauch, nämlich auf der in Gebrauchslage dem Ultraschallkopf zugewandten Seite des Folienschlauches, sowie auf deren abgewandten Seite. Gemäß einer bevorzugten Ausführungsform wird die erste Möglichkeit vorgeschlagen, sodass der erste Kleber und der zweite Kleber auf gegenüberliegenden Seiten des Trägers angeordnet sind. Da der Träger die Schallöffnung abdeckt, wird der zweite Kleber somit ringförmig auf dem Träger aufgebracht sein, um eine dichte Verbindung zu den, die Schallöffnung umgebenden Bereichen des Folienschlauches herzustellen.

Die Verwendung eines Trägers für die Klebefläche und deren Anordnung im Bereich der Schallöffnung bietet den großen Vorteil, dass das Material des Trägers den unterschiedlichen Anforderungen in diesem Bereich angepasst werden kann, als sie etwa an den Folienschlauch gestellt werden. Während der Folienschlauch im Wesentlichen reißfest und widerstandsfähig sein soll, sollte im Durchtrittsbereich der Schallwellen eine gute Durchlässigkeit gegenüber Schallwellen gegeben sein, sowie eine gute Anpassungsfähigkeit an den Schallkopf. Daher besteht bei einer erfindungsgemäßen Ausführungsform nun die Möglichkeit, für den Träger entsprechende Materialien zu wählen, die nicht nur eine zuverlässige Haftung des ersten Klebers sicher stellen, sondern auch gute Eigenschaften hinsichtlich des Schalldurchtrittes aufweisen. Gemäß einer bevorzugten Ausführungsform ist der Träger etwa dünner ausgeführt als der Folienschlauch, und ist aus einem elastomeren Werkstoff gefertigt. Elastomere Werkstoffe können sich bei Zug- und Druckbelastung elastisch verformen. Aufgrund der Elastizität eines elastomeren Werkstoffes passt sich der Träger flexibel dem jeweiligen Schallkopf an. Die geringere Schichtdicke des Trägers erleichtert den Schalldurchtritt.

Des Weiteren ist es denkbar, dass der Träger eine gewölbte Ausgangsform aufweist. Die gewölbte Ausgangsform kann insbesondere der Krümmung des Ultraschallkopfes angepasst werden. Der Träger weist in diesem Fall somit eine dreidimensionale Formgebung auf. Wird der Träger zudem elastisch ausgeführt, ist diese Ausgangsform zwar verformbar, der Träger kehrt aber immer wieder in diese dreidimensionale Ausgangsform zurück. Eine solche Ausführungsform stellt eine dichte Anlage an den Ultraschallkopf sicher, und vermeidet Faltenbildung zur Gänze.

Die Erfindung wird im Folgenden anhand von Ausführungsbeispielen mithilfe der beiliegenden Figuren näher erläutert. Hierbei zeigt die
Fig. 1 eine schematische Darstellung eines erfindungsgemäßen Folienschlauches im Aufbewahrungszustand,
Fig. 2 eine schematische Darstellung einer Anordnung einer Klebefläche auf einem Folienschlauch gemäß dem Stand der Technik,
Fig. 3 eine schematische Darstellung einer ersten Ausführungsform einer erfindungsgemäßen Anordnung einer Klebefläche auf einem Folienschlauch, und die
Fig. 4 eine schematische Darstellung einer bevorzugten Ausführungsform einer erfindungsgemäßen Anordnung einer Klebefläche auf einem Folienschlauch.

Bevor auf die eigentliche Erfindung eingegangen wird, soll zunächst eine Ausführungsform eines Folienschlauches erläutert werden, der im gezeigten Beispiel steril ausgeführt sein soll. Im Aufbewahrungszustand weist der erfindungsgemäße, sackähnliche Folienschlauch eine verkürzende Faltung 2 auf, die einen taschenförmigen Eingriffsbereich 1 bildet. Der taschenförmige Eingriffsbereich 1 wird in seinem Inneren insbesondere durch die innerste, taschenförmig ausgebildete Faltungslage 7 der Faltung 2 gebildet. Diese innerste, taschenförmig ausgebildete Faltungslage 7 der Faltung 2 weist einen geschlossenen Endbereich 6 auf, der die äußeren Faltungslagen überragt. Das Äußere des geschlossenen Endbereiches 6 bildet somit einen Außenflächenabschnitt der Faltung 2. Auf diesem Außenflächenabschnitt ist eine Klebefläche 3 angeordnet, die zur besseren Erkennbarkeit auch von einer sichtbaren Markierung umgrenzt sein kann. Die Klebefläche 3 wird vorzugsweise aus einem sterilisationsbeständigen, ersten Kleber gebildet.

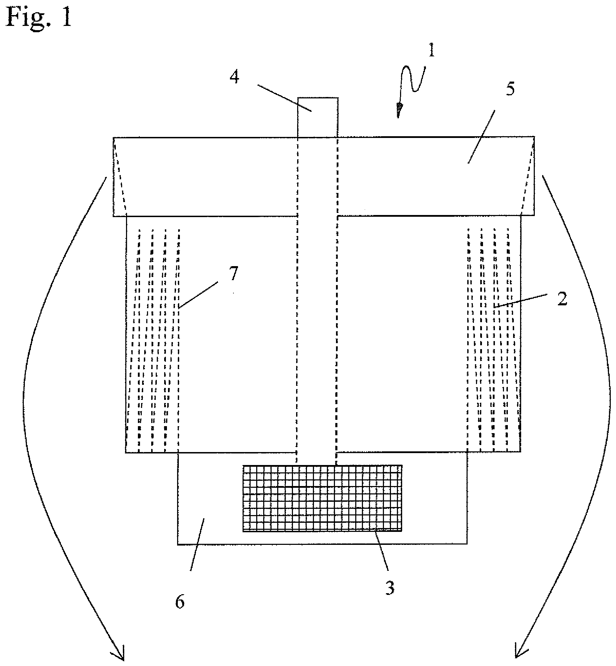

Zur Herstellung der gezeigten Faltung 2 sind unterschiedliche Varianten denkbar. Eine Möglichkeit ist in der Fig. 1 ersichtlich, also eine Faltung 2 mit Faltungslagen, die parallel zur Längsachse des Folienschlauches verlaufen.

Am gegenüberliegenden Ende kann die Faltung 2 in einen Stulp 5 übergehen, der aber nicht Teil der Faltung 2 ist. Die Außenfläche des Stulps 5 bildet nämlich auch im Gebrauchszustand des Folienschlauches einen außen liegenden Abschnitt der Umhüllung, allerdings in patientenfernen Bereichen. Eine allfällige Kontamination des Stulps 5 durch eine nicht sterile Hilfsperson ist somit tolerabel.

Wie des Weiteren der Fig. 1 entnommen werden kann, ist an der innersten, taschenförmigen Faltungslage 7 ein Führungsstreifen 4 befestigt, der bis ins Äußere des taschenförmigen Eingriffsbereiches 1 führt. Der Führungsstreifen 4 erleichtert der behandelnden Person das Auffinden der richtigen Eingriffsöffnung in die innerste, taschenförmige Faltungslage 7, sowie das Lösen allfälliger Verklebungen der innersten, taschenförmigen Faltungslage 7. Vorzugsweise ist der Führungsstreifen 4 am Inneren des geschlossenen Endbereiches 6 befestigt, und als Klebestreifen mit abziehbarer Schutzfolie ausgeführt.

Die Klebefläche 3 am Außenflächenabschnitt der Faltung 2 entspricht in ihrer Ausdehnung im Wesentlichen der Austrittsfläche der Schallwellen am Ultraschallkopf 8 (in der Fig. 1 nicht ersichtlich). Vorzugsweise überragt sie die Austrittsfläche deutlich, um einerseits eine bessere Befestigung am Ultraschallkopf 8 zu ermöglichen, und andererseits für unterschiedliche Ausführungen von Ultraschallgeräten verwendbar zu sein.

Bevor auf die erfindungsgemäße Ausführung der Klebefläche 3 eingegangen wird, soll in weiterer Folge noch die sterile Anwendung eines erfindungsgemäßen Folienschlauches erläutert werden. Im Zuge der Anwendung des erfindungsgemäßen Folienschlauches kann eine unsterile Hilfsperson den an sich steril verpackten Folienschlauch aus der Verpackung entnehmen, und berührt den gefalteten Folienschlauch dabei an seiner Außenseite. Diese Außenseite wird dadurch unsteril. Die Hilfsperson präsentiert den gefalteten Folienschlauch in weiterer Folge der behandelnden Person, etwa indem die Hilfsperson den gefalteten Folienschlauch am Stulp 5 festhält.

Die behandelnde Person schlüpft mit ihrer sterilen Hand in den steril ausgeführten, taschenförmigen Eingriffsbereich 1, die mithilfe des Führungsstreifens 4 leicht aufgefunden werden kann. Die Fingerkuppen dieser Hand befinden sich somit im Bereich des geschlossenen Endabschnittes 6 der innersten, taschenförmig ausgebildeten Faltungslage 7.

Die unsterile Hilfsperson reicht der behandelnden Person in weiterer Folge den Ultraschallkopf 8 (in der Fig. 1 nicht ersichtlich), der etwa pilzförmig ausgeführt ist, wobei sich die Schallfläche in den oberen Bereichen befindet, und die Kabelzuführung an dessen Schaft. Die behandelnde Person drückt mit seinen Fingerkuppen die außen liegende Klebefläche 3 an den Ultraschallkopf 8, wobei die Austrittsfläche der Ultraschallwellen an der Klebefläche 3 befestigt wird. Die unsterile Hilfsperson kann nun den gefalteten Folienschlauch in die in Fig. 1 gezeigte Pfeilrichtung ziehen, also in Richtung des Ultraschallkopfes und seiner Kabelzuführung. Ist der Folienschlauch wie in der Fig. 1 gezeigt ausgeführt, bildet die Außenfläche der Faltung 2 im Gebrauchszustand innen liegende Abschnitte der Umhüllung für das Ultraschallgerät und seiner Kabelzuführung. Die sterilen Innenflächen der Faltung 2 bilden hingegen vollkommen sterile Außenflächenabschnitte der Umhüllung.

Die sterile Hand der behandelnden Person wird im Zuge dieses Umstülpvorganges des Folienschlauches wieder freigegeben, und umfasst weiterhin den Schaft des Ultraschallkopfes 8. Falls der Führungsstreifen 4 als Klebestreifen ausgeführt ist, kann er dazu verwendet werden, den mit dem Folienschlauch abgedeckten Schaft des Ultraschallgeräts knapp unterhalb des Ultraschallkopfes 8 zusätzlich abzudichten. Die Folienschlauchabdeckung sitzt somit sicher und straff am Ultraschallgerät.

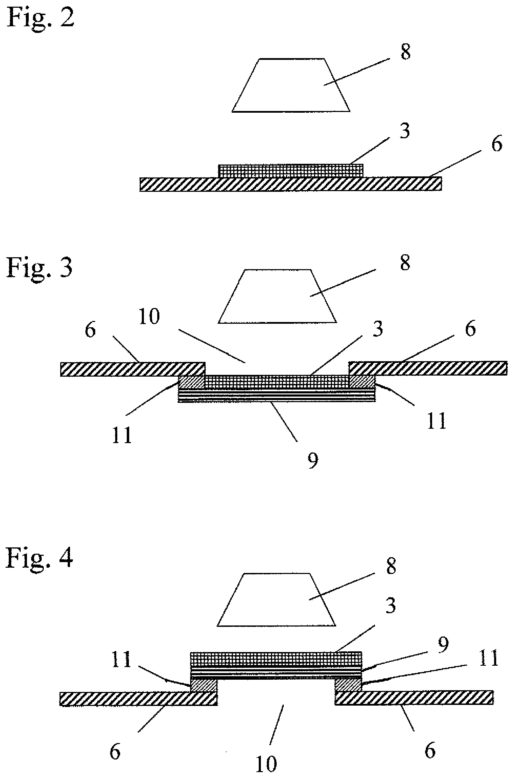

Im Folgenden wird nun auf die Figuren 2 bis 4 eingegangen, wobei die Fig. 2 zunächst eine schematische Darstellung einer Anordnung einer Klebefläche 3 auf einem Folienschlauch gemäß dem Stand der Technik zeigt. Hierbei ist die Klebefläche 3 unmittelbar auf dem Endbereich 6 des Folienschlauches aufgebracht. Bei einer solchen Ausführung wird stets ein Teil des Klebers der Klebefläche 3 beim Ablösen auf dem Ultraschallkopf 8 als Rückstand verbleiben, wodurch eine sorgfältige und mitunter mühsame Reinigung des Ultraschallkopfes 8 erforderlich wird.

Daher wird erfindungsgemäß vorgeschlagen, einen entsprechend ausgeführten Träger 9 für die Klebefläche 3 vorzusehen, wobei sich der für die Klebefläche 3 verwendete, erste Kleber rückstandsfrei vom Ultraschallkopf 8 lösen lässt und vollständig auf dem Träger 9 verbleibt. Mögliche Ausführungsformen sind hierzu in der Fig. 3 und 4 dargestellt, wobei die Schichtdicken vom Folienschlauch, sowie der Träger- und Kleberschichten im Vergleich zum Ultraschallkopf 8 zur besseren Übersichtlichkeit übertrieben dick eingezeichnet wurden. In der Praxis wird die Klebefläche 3 mit einer Schutzfolie (in der Fig. 3 und 4 nicht dargestellt) versehen sein, die vor der Verwendung des Folienschlauches von der Klebefläche 3 abgezogen wird, um die Klebefläche 3 freizugeben.

Die Fig. 3 zeigt eine schematische Darstellung einer ersten Ausführungsform einer erfindungsgemäßen Anordnung einer Klebefläche 3 auf einem Folienschlauch, wobei eine Schallöffnung 10 im Endbereich 6 des Folienschlauches vorgesehen ist, die vom Träger 9 abgedeckt wird. Der Träger 9 ist dabei mit dem Folienschlauch dicht, vorzugsweise keimdicht, verbunden, etwa mithilfe eines zweiten Klebers 11, der die Schallöffnung 10 umgibt. Der Träger 9 kann aber auch aus einem Kunststoff gefertigt und mit dem Folienschlauch verschweißt sein. Im Bereich der Schallöffnung 10 ist die Klebefläche 3 vorgesehen, die dem Ultraschallkopf 8 zugewandt ist.

Eine einfacher und daher kostengünstiger herzustellende Ausführungsform ist in der Fig. 4 dargestellt. Hierbei ist die Klebefläche 3 vollflächig auf der, dem Ultraschallkopf 8 zugewandten Seite des Trägers 9 aufgetragen. Auf der, dem Ultraschallkopf 8 abgewandten Seite des Trägers 9 ist der zweite Kleber 11 ringförmig aufgetragen, und stellt eine dichte, vorzugsweise keimdichte Verbindung mit dem Endbereich 6 des Folienschlauches dar. Alternativ dazu kann der Träger 9 mit dem Folienschlauch auch verschweißt sein. Die Schallöffnung 10 ist somit wiederum vom Träger 9 abgedeckt, der auf diese Weise Teil der keimdichten Biobarriere des Folienschlauches wird. Der erste Kleber der Klebefläche 3 kann dabei im Vergleich zum zweiten Kleber 11 über eine geringere Haftkraft verfügen, um sich leicht vom Ultraschallkopf 8 lösen zu lassen. Der zweite Kleber 11 kann über hohe Haftkraft verfügen, um eine sichere und vorzugsweise keimdichte Verbindung des Trägers 9 zum Folienschlauch sicher zu stellen.

Da der erfindungsgemäße Folienschlauch in der Praxis auch oft als Set mit sonstigen medizinischen Instrumenten, wie etwa Abdeckungen, Nadeln, Katheter, usw., ausgestattet werden soll, stellt auch die überaus leichte Sterilisationsfähigkeit der erfindungsgemäßen Ausführung einen großen Vorteil dar. Eine gängige Art der Sterilisation erfolgt mithilfe von Ethylenoxid im Rahmen der so genannten ETO-Sterilisation, der der erfindungsgemäße Folienschlauch leicht unterzogen werden kann. Vorzugsweise wird für den ersten Kleber der Klebefläche 3, sowie für den zweiten Kleber 11 ein Material verwendet, der gegenüber Ethylenoxid unempfindlich ist, sodass die Klebeeigenschaften aufgrund des Sterilisationsvorganges nicht verändert werden.

Für den Träger 9 sind vorzugsweise entsprechende Materialien zu wählen, die nicht nur eine zuverlässige Haftung des ersten Klebers sicherstellen, sondern auch gute Eigenschaften hinsichtlich des Schalldurchtrittes aufweisen. So kann etwa der Träger 9 in Form einer Trägerfolie aus einem Kunststoff gefertigt und dünner ausgeführt sein als der Folienschlauch, und insbesondere aus einem elastomeren Werkstoff gefertigt sein. Des Weiteren ist auf einen homogenen Auftrag der Klebefläche 3 zu achten, um eine gute Anhaftung des Ultraschallkopfes 8 am Träger 9 unter Vermeidung von Lufteinschlüssen zu gewährleisten. Hierfür ist es vorteilhaft, wenn der Träger 9 eine gewölbte Ausgangsform aufweist, wobei die gewölbte Ausgangsform der Krümmung des Ultraschallkopfes 8 angepasst ist. Der Träger 9 weist in diesem Fall somit eine elastische, dreidimensionale Formgebung auf.

Mithilfe der Erfindung wird somit ein Folienschlauch bereitgestellt, der den Ultraschallkopf 8, insbesondere dessen Schallfläche, bestmöglich schont, und die Verwendung eines Gels zwischen dem Ultraschallkopf 8 und dem Folienschlauch vermeidet. Auch ein oftmaliges Anbringen und Abziehen der Klebefläche 3 zieht die Schallfläche dabei nicht in Mitleidenschaft, da sich die Klebefläche 3 ohne Rückstände vom Ultraschallkopf 8 lösen lässt.

## Patentansprüche

1. Ultraschallkopfabdeckung, die als Folienschlauch sackähnlich zur Umhüllung eines handgeführten Ultraschallkopfes (8) und seiner Kabelzuführung ausgeführt ist und im Aufbewahrungszustand eine verkürzende Faltung (2), die einen taschenförmigen Eingriffsbereich (1) bildet, aufweist, wobei der Folienschlauch eine Klebefläche (3), die von einem ersten Kleber gebildet wird, zur Befestigung des Folienschlauches am Ultraschallkopf (8) aufweist, wobei ein mit dem Folienschlauch dicht verbundener Träger (9) in Form einer Trägerfolie für die Klebefläche (3) vorgesehen ist, der gute Durchlässigkeit gegenüber Schallwellen aufweist, und der Folienschlauch im Bereich der Klebefläche (3) eine Schallöffnung (10) in Form einer Aussparung aufweist, wobei der Träger (9) im Durchtrittsbereich der Schallwellen angeordnet ist und die Schallöffnung (10) abdeckt, und die Klebefläche (3) vollflächig auf der dem Ultraschallkopf (8) zugewandten Seite des Trägers (9) aufgetragen ist.

2. Ultraschallkopfabdeckung nach Anspruch 1, **dadurch gekennzeichnet, dass** der Träger (9) aus einem Kunststoff gefertigt und mit dem Folienschlauch verschweißt ist.

3. Ultraschallkopfabdeckung nach Anspruch 1, **dadurch gekennzeichnet, dass** der Träger (9) mithilfe eines zweiten Klebers (11) mit dem Folienschlauch dicht verbunden ist.

4. Ultraschallkopfabdeckung nach Anspruch 3, **dadurch gekennzeichnet, dass** der erste Kleber der Klebefläche (3) und der zweite Kleber (11) auf gegenüberliegenden Seiten des Trägers (9) angeordnet sind.

5. Ultraschallkopfabdeckung nach einem der Ansprüche 1 bis 4, **dadurch gekennzeichnet, dass** der Träger (9) dünner ausgeführt ist als der Folienschlauch.

6. Ultraschallkopfabdeckung nach einem der Ansprüche 1 bis 5, **dadurch gekennzeichnet, dass** der Träger (9) aus einem elastomeren Werkstoff gefertigt ist.

7. Ultraschallkopfabdeckung nach einem der Ansprüche 1 bis 6, **dadurch gekennzeichnet, dass** der Träger (9) eine gewölbte Ausgangsform aufweist.

## Claims

1. A cover for an ultrasonic transducer which is arranged as film tube in the manner of a bag for enclosing a hand-operated ultrasonic transducer (8) and the cable leads thereof, and which comprises a shortening fold (2) in the stored state, which fold forms a pocket-like engagement area (1), and in which an adhesive surface (3) formed by a first adhesive is provided for attaching the film tube to the ultrasonic transducer (8), wherein a support (9) in form of a support film for the adhesive surface is provided with the support (9) being tightly connected with the film tube cover and having good permeability for sound waves, and the film tube cover comprises a sound opening (10) in form of a recess in the region of the adhesive surface (3), wherein the support (9) is arranged in the passage region of the sound waves and covering the sound opening (10), with the adhesive surface (3) being applied all over to the side of the support (9) to be affixed to the ultrasonic transducer (8).

2. A cover for an ultrasonic transducer according to claim 1, **characterized in that** the support (9) is made of plastic and is welded together with the film tube cover.

3. A cover for an ultrasonic transducer according to claim 1, **characterized in that** the support (9) is tightly connected with the film tube cover by means of a second adhesive (11).

4. A cover for an ultrasonic transducer according to claim 3, **characterized in that** the first adhesive of the adhesive surface (3) and the second adhesive (11) are arranged on opposite sides of the support (9).

5. A cover for an ultrasonic transducer according to one of the claims 1 to 4, **characterized in that** the support (9) is arranged to be thinner than the tube film.

6. A cover for an ultrasonic transducer according to one of the claims 1 to 5, **characterized in that** the support (9) is made of an elastomeric material.

7. A cover for an ultrasonic transducer according to one of the claims 1 to 6, **characterized in that** the support (9) has a curved initial state.

## Revendications

1. Couverture de sonde à ultrasons réalisée comme un tube de film en forme de sac destiné à envelopper une sonde à ultrasons (8) manipulée à la main et son arrivée de câble et formant, dans son état de rangement, un pliage (2) qui la raccourcit, formant une zone de prise (1) en forme de poche, le tube de film présentant une surface de collage (3) qui est formée par un premier adhésif pour la fixation du tube de film sur la sonde à ultrasons (8), dans laquelle il est prévu un support (9) relié de façon étanche au tube de film et prenant la forme d'un film de support pour la surface de collage (3) qui laisse bien passer les ondes sonores, et le tube de film présente dans la zone de la surface de collage (3) une ouverture de passage des ondes sonores (10) prenant la forme d'une découpe, le support (9) étant disposé dans la zone de passage des ondes sonores et recouvrant l'ouverture de passage des ondes sonores (10), et la surface de collage (3) étant appliquée sur toute sa surface sur le côté du support (9) tourné vers la sonde à ultrasons (8).

2. Couverture de sonde à ultrasons selon la revendication 1, **caractérisée en ce que** le support (9) est fait d'une matière plastique et soudé au tube de film.

3. Couverture de sonde à ultrasons selon la revendication 1, **caractérisée en ce que** le support (9) est assemblé de façon étanche au tube de film au moyen d'un deuxième adhésif (11).

4. Couverture de sonde à ultrasons selon la revendication 3, **caractérisée en ce que** le premier adhésif de la surface de collage (3) et le deuxième adhésif (11) sont disposés sur des faces opposées du support (9).

5. Couverture de sonde à ultrasons selon l'une des revendications 1 à 4, **caractérisée en ce que** le support (9) est plus fin que le tube de film.

6. Couverture de sonde à ultrasons selon l'une des revendications 1 à 5, **caractérisée en ce que** le support (9) est fait d'un matériau élastomère.

7. Couverture de sonde à ultrasons selon l'une des revendications 1 à 6, **caractérisée en ce que** le support (9) présente une forme de départ bombée.
